# EUROPEAN PATENT APPLICATION

(11) **EP 1 310 262 A1**
(43) Date of publication of application: **14.05.2003**
(21) Application number: 02024801.9
(22) Date of filing: 07.11.2002
(51) Int. Cl.: A61K 35/78, A61K 7/48

(54) **Topical composition having undifferentiated plant seed cells and method for using same**

(30) Priority: 09.11.2001 US 40242
(71) Applicant: AVON PRODUCTS, INC., New York, NY 10020-1196 (US)
(72) Inventor: Lu, Michelle, Freshmeadows, New York 11365 (US); Menon, Gopinathan K., Wayne, New Jersey 07470 (US); Duggan, Michele, Middletown, New York 10940 (US)
(74) Representative: Dr. Weitzel & Partner

(57) **Abstract**

There is disclosed a topical composition having substantially undifferentiated plant seed cells and, preferably, a pharmaceutically or cosmetically acceptable vehicle. There is also a method for topically applying the composition. There are also methods for delivering a consistent level of an active to skin, nail, lips and/or hair and for enhancing the therapeutic efficacy of a topical composition having substantially undifferentiated plant seed cells.

## Description

### BACKGROUND OF THE INVENTION

### 1. Field of the Invention

The present invention relates to topical compositions having enhanced levels of medicinal, therapeutic or cosmetic active ingredients derived from plant seeds. More particularly, the present invention relates to topical compositions having undifferentiated plant seed cells. Still more particularly, the present invention relates to methods for using the topical compositions.

### 2. Description of the Prior Art

Active ingredients derived from plants and plant seeds have commonly been employed in topical compositions for a myriad of medicinal, therapeutic and cosmetic purposes. Such actives can be obtained from various parts of a plant such as seeds, needles, leaves, roots, bark, cones, stems, rhizomes, callus cells, protoplasts, organs and organ systems and meristems. Active ingredients are incorporated in such compositions in a variety of forms. Such forms include pure or semi-pure component, a solid or liquid extract or derivative, or solid plant matter. Plant matter may be incorporated in a variety of subforms such as whole, minced, ground or crushed.

A problem commonly encountered when using active ingredients derived from plants and plant seeds is the relatively low levels at which they are naturally present. Such low levels frequently require relatively large amounts of plant leaf/tissue or seeds be processed in order to obtain desired or useful quantities of actives. For rare plants or plant seeds, such large amounts may be unavailable or difficult to obtain. Further, expensive extraction techniques, such as solvent extraction, may be necessary to isolate the actives at desired and useful levels.

It would be desirable to have a mechanism for providing enhanced levels of medicinal, cosmetic and therapeutic actives derived from plant seeds. It would be further desirable to have a mechanism for providing such enhanced levels of actives in an economically advantageous manner without expensive extraction techniques.

### SUMMARY OF THE INVENTION

It is an object of the present invention to provide a topical composition that delivers a consistent level of active ingredient produced by substantially undifferentiated plant seed cells.

It another object of the present invention to provide a topical composition having substantially undifferentiated plant seed cells in a pharmaceutically or cosmetically acceptable vehicle.

It is another object of the present invention to provide a topical composition having an enhanced level of a medicinal, therapeutic or cosmetic active ingredient derived from substantially undifferentiated plant seed cells.

It is another object of the present invention to provide a method for topically applying such compositions.

It is another object of the present invention to provide a method for delivering a consistent level of an active ingredient to skin, nail, lips, and/or hair by topical applying a composition having substantially undifferentiated plant seed cells.

It is yet another object of the present invention to provide a method of enhancing the therapeutic efficacy of a topical composition having substantially undifferentiated plant seed cells that is applied to the skin, nail, lips and/or hair.

### DETAILED DESCRIPTION OF THE INVENTION

In the present invention, there is an enhancement in the level and availability of medicinal, therapeutic and cosmetic active ingredients derived from plant sources, particularly plant seeds. A seed typically has a seed coat and an embryo surrounded by endosperm. The plant seed cells are extracted principally from the meristametic tissue of the embryo. Embryonic cells that are located in "non-seed" portion (e.g. rhizomes) of plants, such as iris and ginger, are also contemplated for use in the present invention. The enhancement is partially achieved by providing the total (i.e. entire constituency) of the seed cell. The total seed cell is broken down through breaking or fracturing of cell walls to deliver the broad range of the plant cell constituents. The plant seed cells are then incorporated into a pharmaceutically or cosmetically acceptable vehicle and/or topical composition.

The plant seed cells of a plant have the highest potential to have or to obtain all the plant cell constituents, including any therapeutic, cosmetic or medicinal actives, of that plant. The plant seed cells that have the highest potential are those which are substantially undifferentiated. As used herein, the term "substantially undifferentiated" includes seed cells that have yet to differentiate into specific plant cells, such as leaf cells, root cells, xylum and/or phloem cells, having particular functions or chemical constituents (undifferentiated seed cells); cells that have differentiated to such a limited degree that the seed cells retain a broad range of plant cell constituents that are substantially equivalent to those that are undifferentiated (near undifferentiated seed cells); and seed cells that have de-differentiated into undifferentiated seed cells (de-differentiated seed cells). In other words, the term "substantially undifferentiated" plant seed cells include undifferentiated seed cells, near undifferentiated seed cells and de-differentiated seed cells. Basically, these three types of seed cells are pluripotent and exhibit a broad range of plant cell constituents.

In the present invention, plant seed cells are generally obtained by extracting the seed cells from seed, preferably the seed embryo, and then culturing and homogenizing them *in vitro*. This process significantly increases the amount and number of plant seed cells. Preferably, the seed cells are cultured in a medium that promotes the undifferentiated state. Further preferably, after homogenizing, the seed cells and the plant cell constituents are preserved.

Culturing generally refers to the exposure of the plant seed cells to a suitable nutrient medium. Culturing can be carried out by adding whole seeds or portions thereof, such as the endosperm and/or embryo, to nutrient medium. The cells can be cultured in liquid, semisolid and solid nutrient mediums. The nutrient mediums can be formulated with salts, nutrients, hormones and elicitors to encourage cell growth, cell metabolism and proliferation, cell maintenance, biosynthesis of active ingredients or a combination of the foregoing. Examples of useful medium formulations are set forth in Table 4 of U.S. Patent No. 6,127,181 and Examples 1 to 9 of U.S. Patent No. 5,407,816.

De-differentiating refers to reversing the level of differentiation in differentiated seed cells to an extent that they can be considered undifferentiated or near undifferentiated. The reversal of differentiation generally enhances the range and incidence level of plant cell constituents, including therapeutic, cosmetic and medicinal actives, within the seed cells.

Conditions that can impact de-differentiating include nutrient composition (see above), temperature, light or lack of light, gas headspace composition, operating mode and duration. Light conditions can include natural light (broad wavelength or frequency spectrum), limited or narrow wavelength or frequency spectrum, or no light (darkness). Light can also vary in intensity. Gas headspace composition can vary in oxygen, carbon dioxide and ethylene content (as well as other gases) or lack thereof. Operating mode can vary in process operation such as batch phase, semi-batch phase, continuous phase and multiple phases of the foregoing. For instance, separate phases for cell growth/proliferation and biosynthesis of actives are possible. Duration can vary according to time.

Methods for culturing and de-differentiating plant seed cells are disclosed in U.S. Patent Nos. 5,407,816; 5,885,826; and 6,127,181, which are incorporated herein by reference in their entirety.

Homogenizing generally refers to the breaking or fracturing of the walls of the plant seed cells. Homogenizing enhances the availability of cell seed constituents, including therapeutic, cosmetic and medicinal actives by facilitating their release from the seed cells. Homogenizing can be carried out by mechanically fracturing the cells by any means or method known in the art or by freeze-thaw techniques also known in the art.

Preserving the plant seed cells generally refers to maintaining them under conditions such that the efficacy of desirable actives is not substantially denuded prior to incorporation into the present topical composition. Preservation is not essential to the present invention since seed cells in nutrient medium, i.e. a broth, can be directly topically applied. As a practical matter however, preservation is important to ensure quality and prevent spoilage after culturing and prior to production of a topical composition on a manufacturing scale.

Preservation may be accomplished by any means known in the art such as refrigeration, storage at low pH, i.e. about 4 or less and preferably about 3.6 to about 4.0. Preferably, the seed cells are preserved by maintaining them in a nutrient broth at low pH and refrigerating them.

Advantageously, the substantially undifferentiated plant seed cells are admixed with a cosmetically acceptable vehicle to form the present composition. The cells can be separated from the nutrient medium and admixed with a vehicle, or, more preferably, the cells and the nutrient medium are admixed together in the vehicle. Alternately, the present composition can take the form of the cells in nutrient medium without an additional vehicle, such that the nutrient broth containing the seed cells can be directly topically applied. Further alternatively, the nutrient medium, which invariably contains seed cell constituents even after the seed cells have been separated therefrom, can be directly topically applied by itself or together with a vehicle.

If desired, the seed cells can be stored and provided in a single-use container for easy application and use in manufacturing processes. Alternately, the composition can be spray-dried to a powder form for subsequent admixture with a vehicle to form a composition.

There is another method of the present invention for enhancing the production of a desired plant cell constituent, i.e. an active ingredient. The method comprises inducing substantially undifferentiated plant seed cells under conditions effective to elicit differentiation of the seed cells such that the concentration or production rate of the desired plant cell constituent is enhanced. Inducement or elicitation of differentiation can be carried out according to the techniques and conditions of culturing described above and the in patents disclosed above. Differentiation can also be promoted by control and manipulation of conditions generally described above for de-differentiation except that conditions are controlled and/or manipulated to effect an opposite result. Such differentiated cells may be incorporated into a topical composition along with a cosmetically acceptable vehicle.

Preferred plant seed cells are those cells derived from neem seeds. Neem seed cells are derived from the plant *Azadirachta indica* of the Meliaceae family. Neem seed cells are preferred because they are prolific and produce a broad range of active ingredients. Examples of active ingredients produced by neem seed cells include, but are not limited to, 17-beta-hydroxyazadirdione; 17-epiazadiradione; 1alpha-methoxy-1,2-dihydroazadiradione; 1beta,2beta-diepoxy-azadiradione; 22,23-dihydro-23beta-methoxy-azadiradione, 7-desacetyl-gedunin; azadirachtin; azadirone; epoxyazadiradione; meldenin; meliantriol; nimolicinol; and vepinin. Teachings to topical compositions having neem seed cells and neem seed cell broths are shown in copending application titled Topical Cosmetic Composition With Skin Rejuvenation Benefits, filed November 9, 2001, which is incorporated herein by reference.

Other plant seed cells useful in the present invention other than neem seed cells include, but are not limited to, those from the following plants: Ajuga reptans, caraway, coconut, pomegranate, carrot, cucumber, custard apple, fennel, flax, grape, cumin (particularly black cumin), mangostine, perilla, sunflower and tomato. Embryonic cells from rhizomes are also contemplated.

The plant seed cells may be present in the present topical composition in a wide amount range. The exact amount in the range depends on factors, such as the nature of the active ingredient and the intended use of the topical composition. For the sake of efficiency, the plant seed cells may be incorporated into the composition along with their culture medium in the form of a broth. The broth can be present from about 0.0001 percentage by weight (wt%) to about 50 wt%, preferably from about 0.001 wt% to about 10 wt%, more preferably about 0.01 wt% to about 5 wt%, and most preferably from about 0.1 wt% to about 3 wt% of the total weight of the composition. For purposes of the above disclosed broth concentrations, the broth is 20 wt% plant seed cell, although broths having higher or lower plant seed cell contents are within the scope of the invention.

The identity or nature of any particular active ingredient to be obtained from the plant seed cells, including neem seed cells, is not critical to the present invention. The active ingredient may be useful for any medical, therapeutic, or cosmetic purpose known in the art. The active may be useful in preventing, arresting, ameliorating, diminishing, or treating medical and/or cosmetic conditions of the skin, nail, lips or hair. Such conditions commonly include, but are not limited to, acne, psoriasis, eczema, seborrhea, dermatitis, skin and hair fragility, hair loss, hirsutism, rosacea, pruritis, calluses, warts, corns, dry skin, chapped skin, dandruff, skin blemishes, age spots, hyperpigmentation or hypopigmentation, thinning skin, cellulite, stretch marks, dark circles under the eyes, freckles, yellowing, roughness, keratosis, inflammation, discoloration, skin atrophy, wrinkles, lines, hyperplasia, spider veins (telangectasia), bruising, enlarged pores, fibrosis, sunburn, dermatological aging (chronological aging, hormonal aging and/or actinic aging), viral infections, fungal infections, and bacterial infections. Active ingredients may also be useful in enhancing the general health, vitality and appearance of the skin, nail, lips and hair.

A topical composition having plant seed cells can have active ingredients that improve the aesthetic and/or cosmetic appearance of skin. Such improvements can be manifested in any of the following: reduction in dermatological signs of aging due to, for example, chronological aging, hormonal aging, and photoaging; reduction in skin fragility; reduction in pore size; prevention and/or reversal of loss of collagen and/or elastin; ameliorating the effects of estrogen imbalance; prevention of skin atrophy; prevention and/or reduction in appearance and/or depth of lines and/or wrinkles, including fine lines and/or wrinkles; prevention, reduction and/or treatment of hyperpigmentation; improvement in skin tone, radiance, clarity and/or tautness; prevention, reduction, and amelioration of skin sagging; promotion of anti-oxidant activity; improvement in skin firmness, plumpness, suppleness and/or softness; improvement in procollagen and/or collagen production; improvement in skin texture and/or promotion of retexturization; improvement in skin barrier repair and/or function; improvement in appearance of skin contours; restoration of skin luster and/or brightness; minimization of dermatological signs of fatigue and stress, e.g. skin breakout and/or resistance to environmental stress, e.g. pollution and/or temperature change; replenishment of essential nutrient and/or constituents in the skin decreased by aging and/or menopause; improvement in communication among skin cells; increase in cell proliferation and/or multiplication; increase in skin cell metabolism decreased by aging and/or menopause; retardation of cellular aging; inhibition of enzymes in the skin that accelerate aging of skin cells; minimization of skin dryness and/or improvement in skin moisturization; minimization of skin discoloration, including dark eye circles; promotion and/or acceleration of cell turnover; enhancement of skin thickness; increase in skin elasticity and/or resiliency; enhancement of exfoliation, with or without the use of alpha hydroxy acids or other exfoliants; prevention and reversal of loss of glycosaminoglycans (GAG), collagen and/or elastin; improvement in microcirculation; decrease and/or prevention in cellulite formation; and reduction in acne formation. An example of a seed cell useful in improving microcirculation and decreasing cellulite formation is perilla seed cell. The benefits of perilla seed oil found in perilla seed cells are described in copending U.S. Serial No. 09/521,442, filed March 7, 2000, which is incorporated herein by reference.

In particular, a topical composition having neem seed cells can improve the aesthetic appearance, health and vitality of skin. Such an improvement can be manifested in at least one of the following: prevention and/or reversal of loss of collagen and/or elastin; improvement in skin texture; improvement in skin tone, clarity, and/or tautness; promotion/acceleration of cell turnover; and enhancement of skin thickness.

The present topical composition can be formulated in any suitable product form. Such product forms include, but are not limited to, aerosol spray, cream, dispersion, foam, gel, lotion, mousse, ointment, powder, patch, pomade, solution, pump spray, stick, and towelette.

The present composition preferably includes a vehicle. A useful vehicle is one that is pharmaceutically or cosmetically acceptable. Useful vehicles include, but are not limited to, water, C₁₋₄ alcohols, fatty alcohols, fatty ethers, fatty esters, polyols, glycols, vegetable oils, mineral oils, liposomes, laminar lipid materials, silicone oils, or any mixtures thereof.

Optionally, the present topical composition may include one or more of the following additional ingredients: anesthetics, anti-allergenics, antifungals, antimicrobials, anti-inflammatory agents, antioxidants, antiseptics, chelating agents, colorants, depigmenting agents, emollients, emulsifiers, exfollients, film formers, fragrances, humectants, insect repellents, lubricants, moisturizers, pharmaceutical agents, photostabilizing agents, preservatives, skin protectants, skin penetration enhancers, sunscreens, stabilizers, surfactants, thickeners, viscosity modifiers, vitamins, or any combinations thereof.

The present compositions provide for products, especially cosmetic products, that improve the condition of skin, nail, lips and/or hair. In particular, the compositions of the present invention treat, ameliorate, prevent, inhibit and/or reduce poor skin conditions and the manifestations thereof. With the present invention, it is possible to provide compositions having higher purity, and a more therapeutically specific and standardized supply of active ingredients. Moreover, the present compositions can be formulated to deliver a consistent level of the active ingredient so that a desired cosmetic effect is achieved, especially in batch-to-batch production of commercial products.

The following are non-limiting examples of the present invention. Unless indicated otherwise, all proportions and percentages are by weight.

### Example 1

Humectant (e.g. glycols, glycerols)
0.5-15%
Thickeners (e.g. gums, starches, polymers)
0.1-4%
Chelants I(e.g. disodium EDTA. tetrasodium EDTA)
0.001-0.5%
Preservatives
0.01-2%
Sunscreens (e.g.,Ethylhexylmethoxycinnamate, Benzophephone-3)
0.1-50%
Silicone
0.1-15%
Silica
0.01-10%
Fatty Alcohol/Emulsifiers/Wax/ Fatty Acid
0.5-15%
Emollients
0.1-20%
Plant Seed Cell Broth (e.g. one or more of pomegranate seed cell broth, neem seed cell broth, grape seed cell broth, Ajuga Reptans seed cell broth, coconut seed cell broth, carrot seed cell broth, cucumber seed cell broth, tomato seed cell broth, cumin seed cell broth, fennel seed cell broth, caraway seed cell broth, perilla seed cell broth, mangostine seed cell broth, sunflower seed cell broth, custard apple seed cell broth)
0.0001-50%
Demineralized Water
Q.S.

### Example 2

### Oil-in-Water Emulsion

Humectant (e.g. glycols, glycerols)
0.5-15%
Thickeners (e.g. gums, starches, polymers)
0.1-4%
Chelants I(e.g. disodium EDTA, tetrasodium EDTA)
0.001-0.5%
Preservatives
0.01-2%
Sunscreens (e.g. Ethylhexylmethoxycinnamate, Benzophephone-3)
0.1-50%
Silicone
0.1-15 %
Silica
0.01-10 %
Fatty alcohols/Emulsifers /Wax/ Fatty acids (e.g. Ceteth-20 phosphate/cetearyl alcohol/dicetyl phosphate, Tribehenin PEG-20 Ester, Sodium Dihydroxycetyl phosphate, cetearyl glucoside, cocoglyceride)
0.5-15 %
Emulsion Stabilizers/Viscosity Modifiers (e.g. acrylates/c10-30 alkyl acrylate crosspolymer, acrylate/aminoacrylates/c10-30 alkyl peg-20 itaconate, sodium acrylate/acryloyldimethyl taurate copolymer, acrylates/C₁₀₋₃₀ alkyl acrylate crosspolymer)
0.1-20%
Film Formers(e.g. decene/butene copolymer,
acrylates/octylacrylamide
copolymer, adipic acid/diethylene glycol/glycerin crosspolymer)
0.001-2%
Emollients
0.1-20%
Pomegranate Seed Cell Broth
0.00-20%
Neem Seed Cell Broth
0.01-20%
Grape Seed Cell Broth
0.00-20%
Ajuga Reptans Seed Cell Broth
0.00-20%
Coconut Seed Cell Broth
0.00-20%
Carrot Seed Cell Broth
0.00-20%
Cucumber Seed Cell Broth
0.00-20%
Tomato Seed Cell Broth
0.00-20%
Fennel Seed Cell Broth
0.00-20%
Caraway Seed Cell Broth
0.00-20%
Perilla Seed Cell Broth
0.00-20%
Sunflower Seed Cell Broth
0.00-20%
Custard Apple Seed Cell Broth
0.00-20%
Demineralized Water
Q.S.

### Example 3

### Water/Silicone Emulsion

Sodium PCA 50%
0.1-4%
Sodium Lactate 60%
0.01-10%
Sodium Chloride
0.1-10%
Humectant(Glycerin, Glycols, Glycerols)
0.5-10%,
Ammonium Hydroxide
0.01-10%
Cyclomethicone
0.1-20%
Cyclomethicone/Dimethicone Copolyol
0.1-20%
Emollients(e.g. cetyl octanoate)
0.1-20%
Dimethicone Copolyol/Cyclopentasiloxane
0.1-10%
Pomegranate Seed Cell Broth
0.01-20%
Neem Seed Cell Broth
0.01-20%
Grape Seed Cell Broth
0.00-20%
Ajuga Reptans Seed Cell Broth
0.00-20%
Coconut Seed Cell Broth
0.00-20%
Carrot Seed Cell Broth
0.00-20%
Cucumber Seed Cell Broth
0.00-20%
Tomato Seed Cell Broth
0.00-20%
Fennel Seed Cell Broth
0.00-20%
Caraway Seed Cell Broth
0.00-20%
Perilla Seed Cell Broth
0.00-20%
Sunflower Seed Cell Broth
0.00-20%
Custard Apple Seed Cell Broth
0.00-20%
Demineralized Water
Q.S

### Example 4

### Gel

Carbopol™ (e.g., Carbopol 940 from B.F. Goodrich)
0.01-3%
Glycerin
0.1-30%
Butylene Glycol
0.1-30%
Disodium EDTA
0.01-2%
Methylparaben
0.01-2%
Hydroxyethyl Cellulose
0.01-2%
Corn (Zea Mays) Starch
0.01-10%
C.S. D&C Yellow No.10
0.001-1%
C.S. Fd&C Blue No. 1
0.001-1%
POE (20m) Methyl Glucose Ether
0.01-10%
Dimethyl Polysiloxane
0.01-10%
PEG 50 Shea Butter
0.001-10%
Sodium Hydroxide Solution
0.01-5%
Benzyl Alcohol
0.99000%
Pomegranate Seed Cell Broth
0.01-20%
Neem Seed Cell Broth
0.01-20%
Grape Seed Cell Broth
0.01-20%
Ajuga Reptans Seed Cell Broth
0.00-20%
Coconut Seed Cell Broth
0.00-20%
Carrot Seed Cell Broth
0.00-20%
Cucumber Seed Cell Broth
0.00-20%
Tomato Seed Cell Broth
0.00-20%
Fennel Seed Cell Broth
0.00-20%
Caraway Seed Cell Broth
0.00-20%
Perilla Seed Cell Broth
0.00-20%
Sunflower Seed Cell Broth
0.00-20%
Custard Apple Seed Cell Broth
0.00-20%
Demineralized Water
Q.S

### Example 5

### Cleansing Foam

Humectant (glycerin, butylene glycol)
5-25%
Polyethylene Glycol
0.1-20%
Bentonite
0.1-20%
Stearic Acid
0.1-30%
Myristic Acid
0.1-20%
Cetearyl Alcohol/Ceteareth-20
1.00000%
Potassium Hydroxide 45%
0.1-20%
Preservatives(e.g. benzyl alcohol, 2-phenoxyethanol, benzyl alcohol)
0.1-10%
Pomegranate Seed Cell Broth
0.01-20%
Neem Seed Cell Broth
0.01-20%
Grape Seed Cell Broth
0.01-20%
Ajuga Reptans Seed Cell Broth
0.01-20%
Coconut Seed Cell Broth
0.00-20%
Carrot Seed Cell Broth
0.00-20%
Cucumber Seed Cell Broth
0.00-20%
Tomato Seed Cell Broth
0.00-20%
Fennel Seed Cell Broth
0.00-20%
Caraway Seed Cell Broth
0.00-20%
Perilla Seed Cell Broth
0.00-20%
Sunflower Seed Cell Broth
0.00-20%
Custard Apple Seed Cell Broth
0.00-20%
Demineralized Water
Q.S.

It should be understood that the foregoing description is only illustrative of the present invention. Various alternatives and modifications can be devised by those skilled in the art without departing from the invention. Accordingly, the present invention is intended-to embrace all such alternatives, modifications and variances that fall within the scope of the appended-claims.

## Claims

1. A topical composition, comprising:
substantially undifferentiated plant seed cells; and
a pharmaceutically or cosmetically acceptable vehicle.

2. The composition of claim 1, wherein the plant seed cells, when present in a culture medium, are present in an amount from about 0.0001 wt% to about 50 wt% of the total weight of the composition.

3. The composition of claim 1, wherein the plant seed cells, when present in a culture medium, are present in an amount from about 0.001 wt% to about 10 wt% of the total weight of the composition.

4. The composition of claim 1, wherein the plant seed cells, when present in a culture medium, are present in an amount from about 0.01 wt% to about 5 wt% of the total weight of the composition.

5. The composition of claim 1, wherein the plant seed cells, when present in a culture medium, are present in an amount from about 0.1 wt% to about 3 wt% of the total weight of the composition.

6. The composition of claim 1, wherein the plant seed cells are neem seed cells.

7. The composition of claim 6, wherein the neem seed cells, when present in a culture medium, are present in an amount from about 0.001 wt% to about 10 wt% of the total weight of the composition.

8. The composition of claim 1, wherein the plant seed cells are derived from one or more of plants selected from the group consisting of Ajuga reptans, caraway, coconut, cumin, carrot, custard apple, cucumber, fennel, flax, grape, mangostine, pomegranate, perilla, sunflower and tomato.

9. The composition of claim 1, wherein the vehicle is one or more ingredients selected from the group consisting of water, C₁₋₄ alcohols, fatty alcohols, fatty ethers, fatty esters, polyols, glycols, vegetable oils, mineral oils, liposomes, laminar lipid materials, silicone oils, or any mixtures thereof.

10. The composition of claim 1, wherein the composition is in a product form selected from the group consisting of aerosol spray, cream, dispersion, foam, gel, lotion, mousse, ointment, powder, patch, pomade, solution, pump spray, stick, and towelette.

11. A topical composition, comprising substantially undifferentiated neem seed cells.

12. The composition of claim 11, wherein the need seed cells, when present in a culture medium, are present in an amount from about 0.0001 wt% to about 50 wt% of the total weight of the composition.

13. A method of improving the aesthetic appearance of skin, comprising topically applying a composition having substantially undifferentiated plant seed cells and a pharmaceutically or cosmetically acceptable vehicle.

14. The method of claim 13, wherein the plant seed cells are neem seed cells.

15. The method of claim 13, wherein the plant seed cells, when present in a culture medium, are present in an amount from about 0.0001 wt% to about 50 wt% of the total weight of the composition.

16. The method of claim 13, wherein'the plant seed cells, when present in a culture medium, are present in an amount from about 0.001 wt% to about 10 wt% of the total weight of the composition.

17. The method of claim 13, wherein the improvements are one or more improvements selected from the group consisting of reduction in dermatological signs of aging; reduction in skin fragility; reduction in pore size; prevention and/or reversal of loss of collagen and/or elastin; ameliorating the effects on skin of estrogen imbalance; prevention of skin atrophy; prevention and/or reduction in appearance and/or depth of lines and/or wrinkles; prevention, reduction and/or treatment of hyperpigmentation; improvement in skin tone, radiance, clarity and/or tautness; prevention, reduction, and/or amelioration of skin sagging; promotion of anti-oxidant activity; improvement in skin firmness and/or plumpness and/or suppleness and/or softness; improvement in procollagen and/or collagen production; improvement in skin texture and/or promotion of retexturization; improvement in skin barrier repair and/or function; improvement in appearance of skin contours; restoration of skin luster and/or brightness; minimization of dermatological signs of fatigue and/or stress; resistance to environmental stress; replenishment of ingredients in the skin decreased by aging and/or menopause; improves communication among skin cells; increase in cell proliferation and/or multiplication; increase in skin cell metabolism decreased by aging and/or menopause; retardation of cellular aging; inhibition of enzymes in the skin that accelerate aging of skin cells; minimization of skin dryness and/or improvement in skin moisturization; minimization of skin discoloration, including dark eye circles; promotion and/or acceleration of cell turnover; enhancement of skin thickness; increase in skin elasticity and/or resiliency; and enhancement of exfoliation.

18. The method of claim 13, wherein the improvements are one or more improvements selected from the group consisting of reduction in dermatological signs of aging; prevention and/or reversal of loss of glycosaminoglycans, collagen and/or elastin; improvement in skin texture; prevention or reduction in appearance and/or depth of lines and/or wrinkles; decrease or prevention of cellulite formation; improvement in skin tone, clarity, and/or tautness; restoration of skin luster and/or brightness; and minimization of skin discoloration.

19. A method of improving the aesthetic appearance of skin comprising topically applying a composition having substantially undifferentiated neem seed cells.

20. The method of claim 19, wherein the neem seed cells, when in a culture medium, are present in an amount from about 0.0001 to about 50 wt% of the total weight of the composition.

21. A method of enhancing the therapeutic efficacy of a topical composition for the skin, nail, lips and/or hair, comprising incorporating into the composition substantially undifferentiated plant seed cells.

22. The method of claim 21, wherein the plant seed cell is neem seed cell.

23. A method for enhancing the production of a pre-determined plant cell constituent, comprising
inducing substantially undifferentiated plant seed cells under conditions effective to elicit differentiation of the seed cells such that the concentration or production rate of the pre-determined plant cell constituent is enhanced.

24. A topical composition, comprising the differentiated seed cells of claim 23 and a cosmetically acceptable vehicle.
